# EUROPÄISCHE PATENTSCHRIFT

(11) **EP 3 952 831 B1**
(45) Veröffentlichungstag und Bekanntmachung des Hinweises auf die Patenterteilung: **10.09.2025**
(21) Anmeldenummer: 20705321.6
(22) Anmeldetag: 10.02.2020
(51) Int. Cl.: A61K 8/42, A61K 8/73, A61Q 5/02

(54) **FAST RINSE SHAMPOO**
FAST RINSE SHAMPOO
SHAMPOING À RINÇAGE RAPIDE

(30) Priorität: 08.04.2019 DE 102019204969
(43) Veröffentlichungstag der Anmeldung: 16.02.2022
(73) Patentinhaber: Henkel AG & Co. KGaA, 40589 Düsseldorf (DE)
(72) Erfinder: SCHEELE, Soeren, 25421 Pinneberg (DE); METTE, Manuela, 23923 Kleinfeld (DE); SCHROEDER, Thomas, 22395 Hamburg (DE)
(86) Internationale Anmeldenummer: PCT/EP2020/053346
(87) Internationale Veröffentlichungsnummer: WO 2020/207643

(56) Entgegenhaltungen:
- WO-A1-2016/054451
- US-A1- 2007 292 380

## Beschreibung

Die Anmeldung beschreibt Haarreinigungsmittel auf der Grundlage von Tensiden, kationischen Cassia-Polymeren und Amidoaminen.

Die Anmeldung beschreibt weiterhin die Verwendung dieser Mittel zur Verbesserung der Ausspülbarkeit sowie zur Verbesserung der Pflegeeigenschaften von Haaren.

Mithilfe von Haarshampoos können menschliche Haare und die Kopfhaut gereinigt und von Talg, Stylingmittelrückständen sowie von anderen Verschmutzungen befreit werden.

Bedingt durch die in kosmetischen Haarreinigungsmitteln üblicherweise enthaltenen (meist anionischen) Tenside geht die Haarreinigung stets auch mit einer Entfernung von Lipiden und Proteinen aus den Haaren oder der Kopfhaut einher, wodurch insbesondere bei häufiger Reinigung eine Schädigung der Haarstruktur und/oder ein Austrocknen der Kopfhaut auftreten kann. Schädigungen der Haarstruktur oder der Haarfasern, insbesondere Spliss und/oder Haarbruch, können zudem durch Umwelteinflüsse (wie beispielsweise intensive Sonneneinstrahlung), mechanische Belastungen (wie beispielsweise Kämmen unter Föhnhitze) sowie durch chemische Einflüsse (wie beispielsweise Färben, Verformen oder Glätten der Haare) begünstigt werden.

Zur Verhinderung und/oder Verminderung von Haarschädigungen werden Haarshampoos daher üblicherweise Pflegestoffe wie kationische Pflegepolymere, mineralische oder pflanzliche Öle und/oder Silikone hinzugefügt. Die Pflegestoffe werden auf den Haarfasern abgeschieden und führen bereits ab der ersten Anwendung zu signifikant verbesserten Pflegeeigenschaften wie Kämmbarkeit, Glätte und Weichheit.

Nachteilig an der Inkorporation von Pflegestoffen in Haarshampoos ist oftmals, dass sich dadurch die Ausspüldauer der Shampoos aus den Haaren nach der Reinigung signifikant verlängert. Insbesondere in Ländern mit zunehmender Wasserknappheit spielt die Reduzierung des persönlichen Wasserverbrauchs bei der täglichen Körperhygiene wie auch beim Haarewaschen eine immer größere Rolle, weshalb der Bedarf nach gut reinigenden und pflegenden Shampoos besteht, die sich nach ihrer Anwendung schnell und gründlich wieder ausspülen lassen.

WO 2016/054451 A1 beschreibt konditionierende Haarreinigungsmittel die Haare glänzend und schön machen, keine Silikon Conditioner benutzen und gutes Volumen geben und auswaschbar sind.

Es wurde gefunden, dass zur Verbesserung der Ausspülbarkeit von Shampoos eine Reduzierung des Tensid- und Pflegestoffgehalts in herkömmlichen Zusammensetzungen allein nicht ausreichend ist, denn die Reinigungs- und Pflegeperformance solcher Shampoos mit verringertem Wirkstoffgehalt ist meist nicht mehr zufriedenstellend.

Der vorliegenden Anmeldung lag die Aufgabe zugrunde, Haarreinigungsmittel mit ausgewogener Reinigungs- und Pflegeleistung bereitzustellen, die sich nach ihrer Anwendung schnell und gründlich ausspülen lassen. Eine weitere Aufgabe war, den Wasserverbrauch während der Haarreinigung zu verringern sowie die Abbaubarkeit herkömmlicher Shampoos zu verbessern. Noch eine weitere Aufgabe war die Bereitstellung transparenter Zusammensetzungen.

Es wurde festgestellt, dass durch die Kombination von Tensiden mit kationischen Cassia-Polymeren und spezifischen Amidoaminen Haarpflegeshampoos mit ausgezeichneter Reinigungs- und Pflegewirkung zur Verfügung gestellt werden können.

Aufgrund der stärkeren Ausprägung einer Koazervat-Phase in den Haarreinigungsmitteln lassen diese sich nach ihrer Anwendung sehr gut und schnell aus den Haaren wieder ausspülen und verringern somit die Dauer des gesamten Haarreinigungsvorgangs.

Auf die Verwendung polymerer oder alkoxylierter nichtionischer Stabilisierungsmittel kann für die Herstellung und Lagerung der Shampoos verzichtet werden, so dass bei Bedarf auch Zusammensetzungen hoher Transparenz bereitgestellt werden können.

Die Haarreinigungsmittel verbessern zudem die Kämmbarkeitseigenschaften der Haare, insbesondere die Nasskämmbarkeit. Darüber hinaus bedingt die schnelle und gute Ausspülbarkeit der Haarpflegeshampoos einen geringeren Builtup-Effekt und damit eine Verbesserung des Haargriffs und der Haarfülle.

Ein erster Gegenstand dieser Anmeldung ist ein Haarreinigungsmittel, das enthält
a. mindestens ein Tensid, ausgewählt aus
   ai) 2,5 bis 20 Gew.-% mindestens eines anionischen Tensids und
   aii) 1,0 bis 10 Gew.-% mindestens eines amphoteren und/oder zwitterionischen Tensids,
b. 0,01 bis 1,00 Gew.-% mindestens eines kationischesn Cassia-Polymers und
c. 0,01 bis 2,00 Gew.-% mindestens eines Amidoamins der Formel (I), 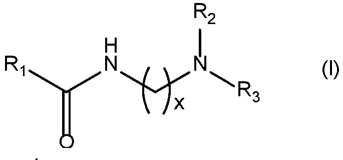 worin
   R₁ für einen gesättigten oder ungesättigten, verzweigten oder unverzweigten Alk(en)ylrest mit 19 bis 30 Kohlenstoffatomen steht,
   R₂ und R₃ unabhängig voneinander für Wasserstoff oder für eine C₁-C₄-Alkylgruppe stehen, und
   x für eine ganze Zahl von 2 bis 6 steht.

Die erfindungsgemäßen Haarreinigungsmittel enthalten die Wirkstoffe a) bis c) bevorzugt in einem kosmetisch akzeptablen Träger. Darunter wird im Rahmen der Erfindung bevorzugt ein wässriger oder wässrig-alkoholischer Träger verstanden.

Der kosmetische Träger enthält bevorzugt mindestens 75 Gew.-%, mehr bevorzugt mindestens 77 Gew.-%, besonders bevorzugt mindestens 78 Gew.-% und insbesondere bevorzugt mindestens 80 Gew.-% Wasser.

Weiterhin kann der kosmetische Träger 0,50 bis 10 Gew.-%, bevorzugt 0,75 bis 9 Gew.-% und insbesondere 1,00 bis 6 Gew.-% mindestens eines Alkohols enthalten.

Geeignete Alkohole sind beispielsweise Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Besonders bevorzugt sind Polyole, denn es wurde gefunden, dass diese die Pflegewirkung des erfindungsgemäßen Wirkstoffkomplexes weiter unterstützen, ohne die Schaumeigenschaften der Mittel negativ zu beeinflussen. Darüber hinaus trägt ein gewisser Polyolgehalt zur besseren Solubilisierung von Ölen bei, die den Haarreinigungsmitteln gegebenenfalls hinzugefügt werden können, wodurch auf polymere Stabilisierungsmittel und/oder alkoxylierte nichtionische Emulgatoren gänzlich verzichtet werden kann.

Ein insbesondere bevorzugtes Polyol ist Glycerin, das in den erfindungsgemäßen Mitteln in den zuvor genannten Mengen eingesetzt werden kann.

Die erfindungsgemäßen Haarreinigungsmittel enthalten als ersten wesentlichen Inhaltsstoff mindestens ein Tensid a), wie vorhergehend beschrieben.

Die Milde sowie die guten Schaumeigenschaften der erfindungsgemäßen Haarreinigungsmittel können in besonderem Maße durch die sorgfältige Auswahl der Tensidmengen und/oder der Tensidtypen gesteuert werden.

So enthalten die erfindungsgemäßen Haarreinigungsmittel vorzugsweise mindestens ein anionisches Tensid, welches für die Erzeugung zufriedenstellender Schaummengen und Schaumeigenschaften wesentlich ist. Für die Erzielung einer optimalen Balance zwischen Milde und Schaumeigenschaften der erfindungsgemäßen Haarreinigungsmittel hat sich eine Mischung aus mindestens einem anionischen Tensid und mindestens einem milden Co-Tensid herausgestellt, welches vorzugsweise aus amphoteren und/oder zwitterionischen und/oder nichtionischen Tensiden ausgewählt sein kann.

Die Kombination des oder der Tensids(e) a) mit den Komponenten b) und c) wiederum ermöglicht eine Steigerung der Haarpflege sowie die gründliche und schnelle Ausspülbarkeit des gebildeten Tensidschaums.

Es enthalten die erfindungsgemäßen Haarreinigungsmittel daher (bezogen auf das Gewicht des gesamten Mittels)
- 2,50 bis 20,00 Gew.-% mindestens eines anionischen Tensids ai) und/oder
- 1,00 bis 10,00 Gew.-% mindestens eines amphoteren und/oder zwitterionischen Tensids aii),
besonders bevorzugt mindestens ein anionisches Tensid ai) und mindestens ein amphoteres und/oder zwitterionisches Tensid aii) in den zuvor genannten Mengen.

Zu den geeigneten anionischen Tensidtypen ai), die in den erfindungsgemäßen Haarreinigungsmitteln eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe (Sarcosinat-Tenside),
- Acyltauride mit 8 bis 24 C-Atomen in der Acylgruppe (Taurat-Tenside),
- Acylisethionate mit 8 bis 24 C-Atomen in der Acylgruppe (Isethionat-Tenside),
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen (Sulfosuccinat-Tenside),
- Alpha-Olefinsulfonate mit 8 bis 24 C-Atomen (alpha-Olefinsulfonat-Tenside),
- Alkylsulfate und/oder Alkylethersulfatsalze der Formel R-(OCH₂-CH₂)ₙ-O-SO₃X, in der R bevorzugt eine geradkettige oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 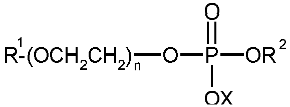 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R ⁴R ⁵R⁶ steht, mit R³ bis R⁶ unabhängig voneinander stehend für einen C₁ bis C₄ -Kohlenwasserstoffrest.

Besonders bevorzugt sind Alkylsulfat- und/oder Alkylethersulfatsalze, (Salze von) Ethercarbonsäuren, Isethionate, Taurate und/oder Alpha-Olefinsulfonate, insbesondere Alkylsulfat- und/oder Alkylethersulfatsalze.

Das oder die anionische Tensid(e) ai) wird (werden) in den erfindungsgemäßen Haarreinigungsmitteln (bezogen auf das Gewicht des gesamten Mittels) bevorzugt in einer Menge von 2,50 bis 20,00 Gew.-%, mehr bevorzugt von 3,00 bis 18,00 Gew.-%, besonders bevorzugt von 4,00 bis 16,00 Gew.-%, ganz besonders bevorzugt von 5,00 bis 14,00 Gew.-% und insbesondere von 6,00 bis 12,50 Gew.-% eingesetzt.

In einer ganz bevorzugten Ausführungsform wird als anionisches Tensid ai) mindestens ein Alkylsulfat- und/oder Alkylethersulfatsalz, Ethercarbonsäure(salz), Isethionat, Taurat und/oder Alpha-Olefinsulfonat, insbesondere ein Alkylsulfat- und/oder Alkylethersulfatsalz, in den zuvor genannten Mengen in den erfindungsgemäßen Haarreinigungsmitteln eingesetzt.

Zu den geeigneten amphoteren und/oder zwitterionischen Tensidtypen aii), die in den erfindungsgemäßen Haarreinigungsmitteln eingesetzt werden können, zählen beispielsweise eine oder mehrere Verbindungen der nachfolgenden Formeln (i) bis (vii). Darin steht der Rest R vorzugsweise für einen geradkettigen oder verzweigten, gesättigten oder ein- oder mehrfach ungesättigten Alkyl- oder Alkenylrest mit 7 bis 23 Kohlenstoffatomen (Formeln (i) und (ii)) oder für einen geradkettigen oder verzweigten, gesättigten oder ein- oder mehrfach ungesättigten Alkyl- oder Alkenylrest mit 8 bis 24 Kohlenstoffatomen (Formeln (iii) bis (vii)):

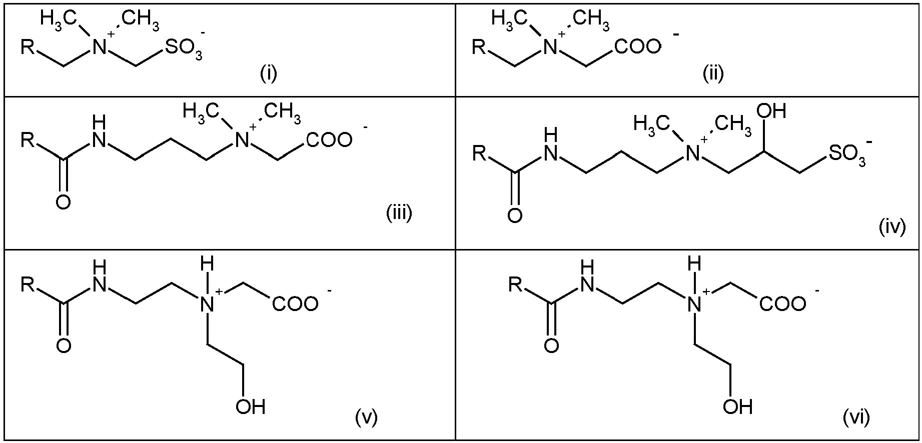

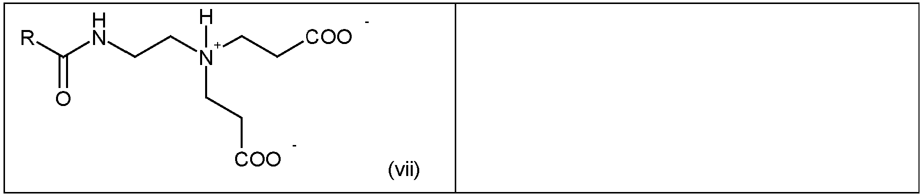

Bevorzugte amphotere Tenside der zuvor genannten Formeln (i) bis (vii) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 8 bis 16 C-Atomen. Besonders bevorzugt sind amphotere Tenside b), bei denen sich der Rest R von Kokosfett ableitet. Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren Tenside.

Insbesondere bevorzugt sind Tenside mit den INCI-Bezeichnungen Cocamidopropylbetain, Lauramidopropylbetain, Cocoampho(di)acetate und/oder Lauroapho(di)acetate.

Das oder die amphotere(n) und/oder zwitterionische(n) Co-Tensid(e) aii) wird (werden) in den erfindungsgemäßen Haarreinigungsmitteln (bezogen auf deren Gesamtgewicht) vorzugsweise in einer Menge von 0,10 bis 10,00 Gew.-%, mehr bevorzugt von 0,50 bis 8,00 Gew.-%, besonders bevorzugt von 1,00 bis 6,00 Gew.-% und insbesondere von 2,00 bis 5,00 Gew.-% eingesetzt.

Besonders bevorzugte anionische Tenside im Sinne der vorliegenden Erfindung sind aufgrund ihrer hervorragenden Schaumeigenschaften Alkyl(ether)sulfate. Diese werden - zur Steigerung der Milde - vorzugsweise mit Cocamidopropylbetain und/oder Cocoampho(di)acetate als Co-Tensid kombiniert.

Für einige Anwendungsformen - beispielsweise für die Reinigung und Pflege stark vorgeschädigter Haare und/oder besonders feiner Haare und/oder von Baby- oder Kleinkinderhaaren - kann es von Vorteil sein, auf die Verwendung von Sulfattensiden zu verzichten.

Ein Verzicht auf Sulfattenside geht oftmals mit einer dramatischen Verschlechterung der Schaumeigenschaften (Quantität und Qualität) einher, so dass die Auswahl geeigneter Tenside sehr mühsam ist. Die Einarbeitung ölbasierter Pflegestoffe in Haarreinigungsmittel wirkt sich zudem negativ auf die Stabilität, die Viskosität und die Schaumeigenschaften der Mittel aus.

Es wurde gefunden, dass pflegende Haarreinigungsmittel mit guten Schaumeigenschaften und hervorragendem Pflegepotential hergestellt werden können, wenn als anionische, sulfatfreie Tensidbasis mindestens ein Tensid aus der Gruppe der anionischen Taurat-Tenside und/oder Isethionat-Tenside und/oder alpha-Olefinsulfonat-Tenside gewählt wird. Vorzugsweise wird mindestens eines dieser Tenside mit Cocamidopropylbetain und/oder Cocoampho(di)acetate als Co-Tensid kombiniert.

Geeignete nichtionische Tenside für die Verwendung als Co-Tensid sind beispielsweise
- Aminoxide, die ausgewählt sein können aus Verbindungen der allgemeinen Formeln (I) oder 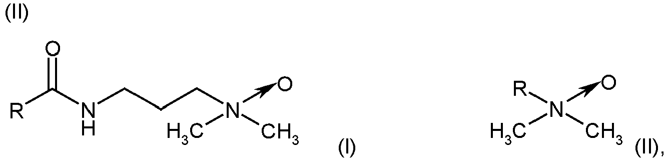
   in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.
   Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (I) oder (II).
- Fettsäurealkanolamide der nachfolgenden allgemeinen Formel, 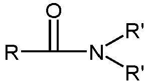 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Gemische aus Alkyl-(oligo)-glucosiden und Fettalkoholen zum Beispiel das im Handel erhältliche Produkt Montanov^{®}68,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden,
- Alkyl(oligo)glycoside. Geeignete Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.

Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann. Besonders bevorzugte Alkyl(oligo)glycoside weisen einen Oligomerisierungsgrad zwischen 1,2 und 1,5 auf. Der Rest R steht bevorzugt für mindestens einen Alkyl- und/oder Alkenylrest mit 4 bis 24 C-Atomen.

Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Besonders bevorzugte nichtionische Tenside, die in den erfindungsgemäßen Haarreinigungsmitteln als Co-Tensid enthalten sein können, sind Fettsäurealkanolamide, Alkyl(oligo)glucoside und Aminoxide. Insbesondere bevorzugt sind Alkyl(oligo)glucoside, welche beispielsweise auch als Mischung mit Glycerylestern wie beispielsweise Glycerylmonooleat (GMO) in den erfindungsgemäßen Mitteln eingesetzt werden können. Entsprechende Produkte sind im Handel beispielsweise unter der Bezeichnung Lamesoft^{®} PO 65 von der Firma BASF erhältlich.

Das oder die nichtionische(n) Co-Tensid(e) wird (werden) in den erfindungsgemäßen Haarreinigungsmitteln (bezogen auf deren Gesamtgewicht) vorzugsweise in einer Menge von 0,05 bis 3,00 Gew.-%, mehr bevorzugt von 0,10 bis 2,50 Gew.-%, besonders bevorzugt von 0,20 bis 2,25 Gew.-% und insbesondere von 0,30 bis 2,00 Gew.-% eingesetzt.

Insbesondere bevorzugt wird in den erfindungsgemäßen Haarreinigungsmitteln ein Alkyl(oligo)glucosid in den zuvor genannten Mengen eingesetzt.

Eine zweite wesentliche Komponente in den erfindungsgemäßen Haarreinigungsmitteln ist ein kationisches Cassia-Polymer b). Ein Gehalt dieser spezifischen Polymertypen in der erfindungsgemäßen Wirkstoffmischung ist nicht nur für die Verbesserung der Haarpflegeeigenschaften von Vorteil, sondern es wurde zudem gefunden, dass sie - im Gegensatz zu vielen anderen kationischen Polymeren - insbesondere bei regelmäßiger Anwendung keinen Überkonditionierungseffekt und/oder einen glitschigen Haargriff fördern.

Üblicherweise werden kationische Polymere in Haarshampoos eingesetzt, um die Abscheidung von Ölen - insbesondere Silikonölen - auf den Haarfasern zu steigern. Dadurch lässt sich nachweislich eine signifikante Verbesserung aller relevanter Haarpflegeparameter erzielen. Bei häufiger Anwendung solcher Shampoos tritt jedoch oftmals der unerwünschte, sogenannte Builtup-Effekt auf, welcher dadurch zustande kommt, dass eine zu große Menge an Pflegestoffen dauerhaft auf den Haaren verbleibt, wodurch diese schwer, glitschig und unansehnlich werden. Darüber hinaus werden von den Verbrauchern zunehmend natürlichere und nachhaltigere Produkte gewünscht, in denen optimalerweise keine Silikone verwendet werden.

Es muss demnach eine Balance zwischen sehr gutem Abscheidevermögen von Pflegestoffen und guter Ausspülbarkeit überschüssiger Pflegestoffe gefunden werden.

Es wurde gefunden, dass die kationischen Cassia-Polymere b) in Kombination mit Tensiden a) und Amidoaminen c) besser als andere, üblicherweise in Haarpflegeshampoos verwendeten kationischen Polymere geeignet sind, Haarpflegeöle (auch Nicht-Silikonöle) auf den Haaren abzuscheiden. Dadurch kann die Menge der Öle reduziert, die Ausspülbarkeit verbessert und ein Überkonditionierungseffekt verhindert werden.

Erfindungsgemäß geeignete Cassia-Polymere b) können aus in tropischen Zonen der Erde (wie beispielsweise in Indien) beheimateten und dort wildwachsenden Cassiasamen, -blättern und/oder -bohnen gewonnen werden. Es handelt sich dabei um Galactomannanpolymere, die aus Kombinationen von Mannose- und Galactose-Monomeren in unterschiedlichen Gehalten bestehen. Darin sind die Mannoseeinheiten untereinander über ß(1-4)-glykosidische Bindungen verbunden; die Galactoseeinheiten über α(1-6)-Bindungen. Das Verhältnis von Mannose- zu Galactose-Monomeren unterscheidet sich je nach Art und Herkunft der Pflanze sowie nach der Temperatur, bei der sie gewachsen ist.

Bei Guar Gum als Beispiel für ein Galactomannan beträgt das Mannose-Galactose-Verhältnis üblicherweise 2:1 (entspricht 2 Monomeren Mannose zu einem Monomer Galactose).

Bei den erfindungsgemäßen, Nicht-Guar-Galactomannanen aus beispielsweise in Indien beheimateten Cassiasamen, -blättern und/oder -bohnen beträgt das Mannose-Galactose-Verhältnis >2:1, vorzugsweise >2,5:1 und insbesondere >3:1. Die Bestimmung des Mannose-Galactose-Verhältnisses ist im Stand der Technik bekannt und basiert üblicherweise auf der Bestimmung des Galactosegehalts in dem Galactomannan.

Erfindungsgemäß besonders geeignete, Nicht-Guar Galactomannane aus beispielsweise in Indien beheimateten Cassiasamen, -blättern und/oder -bohnen werden kationisch modifiziert, indem vorzugsweise die Hydroxylgruppen der Galactomannanpolymere mit reaktiven quaternären Ammoniumverbindungen zur Reaktion gebracht werden. Als geeignete quaternäre Ammoniumverbindungen kommen vorzugsweise Verbindungen der nachfolgenden Formel

N⁺(R¹R²R³R⁴) X⁻

in Frage, in der
R¹, R² und R³ für Methyl- oder Ethylgruppen und
R⁴ für eine Epoxy-R⁵- oder eine Halohydringruppe Y-CH₂-CH(OH)-R⁵- steht, in der R⁵ eine C₁-C₃-Alkylengruppe, Y ein Halogenid und X ein Anion wie Cl⁻, Br, I⁻ oder HSO4⁻ ist.

Ganz besonders geeignete kationische Cassia-Polymere b) im Sinne der vorliegenden Erfindung entsprechen der Formel

R-O-CH₂-CH(OH)-R⁵-N⁺(R¹R²R³) X⁻,

worin R das Galactomannan aus beispielsweise in Indien beheimateten Cassiasamen, -blättern und/oder -bohnen ist, und die anderen Reste dieselbe Bedeutung wie oben haben.

Die besondere Struktur der Galactomannane aus Cassia ermöglicht einen höheren Grad der kationischen Modifizierung (höherer kationischer Substitutionsgrad), wodurch in den erfindungsgemäßen Shampoos eine bessere Koazervatbildung und eine bessere Pflegeleistung erzielt wird (gegenüber Shampoos, die übliche kationische Polymere wie Polyquaternium-10 und/oder Guar Hydroxypropyltrimonium Chloride enthalten).

In einer besonders bevorzugten Ausführungsform enthalten die erfindungsgemäßen Haarreinigungsmittel kationische Cassia-Polymere b), die mit kationischen Hydroxy-C₁-C₃-alkyl-trialkylammonium-Gruppen, insbesondere mit Hydroxypropyltrimethylammoniumgruppen, kationisch modifiziert wurden.

Innerhalb dieser Ausführungsform sind insbesondere unter der INCI-Bezeichnung Cassia Hydroxypropyltrimonium Chloride bekannte und im Handel erhältliche kationische Polymere bevorzugt.

Diese weisen vorzugsweise ein mittleres Molgewicht im Bereich von 10,000 bis 3,000,000 (bestimmbar beispielsweise mittels Gelpermeationschromatographie) auf. Weiterhin weisen sie vorzugsweise eine kationische Ladungsdichte im Bereich von >1 bis 7 meq/g, mehr bevorzugt >1,2 bis 6 meq/g, besonders bevorzugt >1,4 bis 5 meq/g und insbesondere >1,6 bis 4 meq/g auf.

Das Handelsprodukt Clearhance^{®} C, erhältlich von der Firma Ashland, ist ein Beispiel für ein erfindungsgemäß ganz besonders bevorzugtes kationisches Cassia-Polymer b).

Erfindungsgemäß geeignete kationische Cassia-Polymere b) werden in den erfindungsgemäßen Haarreinigungsmitteln (bezogen auf deren Gesamtgewicht) bevorzugt in Mengen von 0,01 bis 1,00 Gew.-%, mehr bevorzugt 0,02 bis 0,90 Gew.-%, besonders bevorzugt 0,03 bis 0,80 Gew.-%, ganz besonders bevorzugt 0,04 bis 0,70 Gew.-% und insbesondere 0,05 bis 0,60 Gew.-% eingesetzt. Insbesondere bevorzugt werden unter der INCI-Bezeichnung Cassia Hydroxypropyltrimonium Chloride bekannte Cassia-Polymere b) in den zuvor genannten Mengen in den erfindungsgemäßen Haarreinigungsmitteln eingesetzt.

Eine dritte wesentliche Komponente der Pflegewirkstoffmischung in den erfindungsgemäßen Haarreinigungsmitteln ist ein Amidoamin c) der Formel (I), 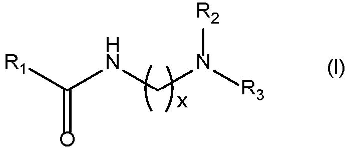 worin

R₁ für einen gesättigten oder ungesättigten, verzweigten oder unverzweigten Alkylrest mit 19 bis 30 Kohlenstoffatomen steht,

R₂ und R₃ unabhängig voneinander für Wasserstoff oder für eine C₁-C₄-Alkylgruppe stehen, und x für eine ganze Zahl von 2 bis 6 steht.

Amidoamine c) der Formel (I) verleihen Haaren ein ausgesprochen weiches, geschmeidiges Gefühl, ohne es zu beschweren. In Kombination mit den spezifischen kationischen Cassia-Polymeren b) tragen sie in Haarreinigungsmitteln damit zu einer signifikanten Erhöhung des Haarpflegeeffekts bei, ohne die Haarfülle negativ zu beeinträchtigen. Dazu trägt auch bei, dass sich die Wirkstoffmischung b) und c) nach der Anwendung der Mittel und der üblichen Einwirkungszeit schnell und gründlich aus den Haaren wieder ausspülen lassen.

Es wurde gefunden, dass insbesondere Amidoamine c) mit einem mehr als 19 C-Atome, vorzugsweise mehr als 20 C-Atome, umfassenden Alkylrest (R₁) die Kämmbarkeit zudem verbessern.

Besonders geeignet sind daher Amidoamine c) gemäß Formel (I), worin
- R₁ für einen Alkenylrest mit 20 bis 24 Kohlenstoffatomen steht,
- R₂ und R₃ für Methylgruppen stehen, und
- x für die Zahlen 2, 3 oder 4 steht.

Insbesondere bevorzugt für die Verwendung in den erfindungsgemäßen Haarreinigungsmitteln sind Amidoamine c) gemäß Formel (I), die aus unter der INCI-Bezeichnung Behenamidopropyl Dimethylamine bekannten Amidoaminen ausgewählt sind.

Solche Amidoamine sind im Handel von verschiedenen Anbietern erhältlich und für die Verwendung in den erfindungsgemäßen Mitteln besonders geeignet; beispielsweise Amidet^{®} APA-22 von der Firma Kao Corporation.

Das oder die Amidoamin(e) c) wird (werden) in den erfindungsgemäßen Haarreinigungsmitteln (bezogen auf das Gewicht des gesamten Mittels) bevorzugt in einer Menge von 0,01 bis 2,00 Gew.-%, mehr bevorzugt von 0,025 bis 1,75 Gew.-%, besonders bevorzugt von 0,05 bis 1,50 Gew.-%, ganz besonders bevorzugt von 0,10 bis 1,25 Gew.-% und insbesondere von 0,15 bis 1,00 Gew.-% eingesetzt.

Zur weiteren Steigerung der Haarpflegeeigenschaften der erfindungsgemäßen Haarreinigungsmittel ist es weiterhin von Vorteil, wenn diese zusätzlich zu den Pflegestoffen b) und c) mindestens ein natürliches, mineralisches und/oder synthetisches Öl enthalten.

Eine weitere bevorzugte Ausführungsform der Erfindung ist deshalb dadurch gekennzeichnet, dass die Haarreinigungsmittel zusätzlich mindestens ein natürliches, mineralisches und/oder synthetisches Öl d) in einem Gewichtsanteil von 0,1 bis 10 Gew.-% am Gesamtgewicht des Haarreinigungsmittels enthalten.

Innerhalb dieser Ausführungsform ist es besonders bevorzugt, wenn eine Mischung von Ölen d) in den erfindungsgemäßen Haarreinigungsmitteln eingesetzt wird, vorzugsweise eine Mischung aus
(i) mindestens einem Mono- und/oder Diester von Glycerin und einer gesättigten oder ungesättigten, verzweigten oder unverzweigten Carbonsäure mit 18 bis 30 Kohlenstoffatomen, und
(ii) mindestens einem pflanzliches Öl.

Besonders geeignete Mono- und/oder Diester d)(i) können ausgewählt sein aus Mono- und/oder Diestern von Glycerin und ungesättigten Carbonsäuren mit 18 bis 24 Kohlenstoffatomen , ganz besonders bevorzugt aus Mono- und/oder Diester d)(i), die sich sehr gut und ohne polymere und/oder alkoxylierte nichtionische Hilfsstoffe in den erfindungsgemäßen Haarreinigungsmitteln lösen und/oder solubilisieren lassen, so dass klare Rezepturen erhalten werden können. Insbesondere bevorzugt sind Mono- und/oder Diester d)(i) von Glycerin und Petroselinsäure, Ölsäure, Gadoleinsäure, Gondosäure, Cetoleinsäure, Erucasäure, Linolsäure und oder Linolensäure, da sie zudem aus natürlichen (pflanzlichen) Quellen zugänglich sind und gut biologisch abbaubar sind.

Insbesondere bevorzugt ist ein Mono- und/oder Diester d)(i) von Glycerin und Ölsäure (INCI-Bezeichnung: Glyceryl Oleate), denn diese Ester weisen hervorragende rückfettende Eigenschaften auf und unterstützen die gute Kämmbarkeit und den Griff der Haare.

Der oder die Mono- und/oder Diester von Glycerin und ungesättigten Carbonsäuren mit 18 bis 24 Kohlenstoffatomen d)(i) wird (werden) in den erfindungsgemäßen Haarreinigungsmitteln (bezogen auf das Gewicht des gesamten Mittels) bevorzugt in einer Menge von 0,10 bis 5,00 Gew.-%, mehr bevorzugt von 0,15 bis 4,50 Gew.-%, besonders bevorzugt von 0,20 bis 4,00 Gew.-%, ganz besonders bevorzugt von 0,25 bis 3,50 Gew.-% und insbesondere von 0,30 bis 3,00 Gew.-% eingesetzt.

In einer bevorzugten Ausführungsform wird ein Mono- und/oder Diester d)(i) von Glycerin und Ölsäure (INCI-Bezeichnung: Glyceryl Oleate) in den zuvor genannten Mengen in den erfindungsgemäßen Haarreinigungsmitteln eingesetzt.

Geeignete Öle d)(ii) sind beispielsweise Amaranthsamenöl, Aprikosenkernöl, Arganöl, Avocadoöl, Babassuöl, Baumwollsaatöl, Borretschsamenöl, Camelinaöl, Canolaöl, Cranberryöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Kokosnussöl, Kürbiskernöl, Leinöl, Macadamianussöl, Maiskeimöl, Malvenöl, Mandelöl, Mangokernöl, Marulaöl, Mohnöl, Nachtkerzenöl, Olivenöl, Palmöl, Palmkernöl, Pfirsichkernöl, Rambutanöl, Rapssamenöl, Reiskleieöl, Rizinusöl, Sacha Inchi Öl, Safloröl, Sanddornfruchtfleischöl, Sanddornkernöl, Sasanquaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl und/oder Wildrosenöl. Bevorzugt sind Arganöl, Avocadoöl, Jojobaöl, Mandelöl, Rapsöl und/oder Sonnenblumenöl. Insbesondere bevorzugt ist Arganöl.

Es wurde gefunden, dass durch die erfindungsgemäße Wirkstoffmischung, insbesondere durch die darin enthaltenen kationischen Cassia-Polymere b), Ester d)(i) und/oder Pflanzenöle d)(ii) - auch in geringen Konzentrationen eingesetzt - besonders gut auf den Haarfasern abgeschieden werden können, und die antistatischen Eigenschaften sowie den Glanz der behandelten Haare verbessern. Durch die gleichzeitig gute und gründliche Ausspülbarkeit der Zusammensetzungen kommt es auch bei regelmäßiger Anwendung der erfindungsgemäßen Haarshampoos zu keiner Überkonditionierung.

Das oder die pflanzliche(n) Öl(e) d)(ii) wird (werden) in den erfindungsgemäßen Haarreinigungsmitteln (bezogen auf das Gewicht des gesamten Mittels) bevorzugt in einer Menge von 0,005 bis 1,00 Gew.-%, mehr bevorzugt von 0,01 bis 0,90 Gew.-%, besonders bevorzugt von 0,015 bis 0,80 Gew.-%, ganz besonders bevorzugt von 0,02 bis 0,70 Gew.-% und insbesondere von 0,025 bis 0,60 Gew.-% eingesetzt.

Für eine sehr gute (Kopf)hautverträglichkeit der erfindungsgemäßen Haarreinigungsmittel ist es von Vorteil, wenn diese einen leicht aciden pH-Wert aufweisen.

Es wurde gefunden, dass erfindungsgemäße Haarreinigungsmittel in einem pH-Bereich von 4,0 bis 6,0 besonders gute Hautverträglichkeit und Milde aufweisen.

In einer weiteren bevorzugten Ausführungsform weisen die erfindungsgemäßen Haarreinigungsmittel daher einen pH-Wert im Bereich von 4,0 bis 6,0, mehr bevorzugt von 4,2 bis 5,9 und insbesondere bevorzugt von 4,5 - 5,8 auf.

In einer weiteren bevorzugten Ausführungsform können die erfindungsgemäßen Haarreinigungsmittel zur Einstellung des pH-Wertes, aber gleichzeitig auch zur Unterstützung der haarpflegenden Eigenschaften, zusätzlich mindestens eine Hydroxycarbonsäure in einer bevorzugten Menge (bezogen auf das Gewicht des gesamten Mittels) von 0,10 bis 5,00 Gew.-%, mehr bevorzugt von 0,20 bis 4,00 Gew.-%, besonders bevorzugt von 0,30 bis 3,00 Gew.-% und insbesondere von 0,40 bis 2,00 Gew.-% enthalten.

Unter geeigneten Hydroxycarbonsäuren werden vorzugsweise alpha-Hydroxycarbonsäuren und insbesondere - beispielsweise in Fruchtsäuren - natürlich vorkommende alpha-Hydroxycarbonsäuren verstanden. Darunter fallen beispielsweise Äpfelsäure, Zitronensäure, Glycolsäure, Isozitronensäure, Mandelsäure, Milchsäure, Tartronsäure und/oder Weinsäure. Unter geeigneten Hydroxycarbonsäuren werden weiterhin die Dicarbonsäuren Glutaminsäure und/oder Bernsteinsäure verstanden.

Besonders bevorzugt sind Zitronensäure, Milchsäure, Glutaminsäure und/oder Bernsteinsäure.

Die erfindungsgemäßen Haarreinigungsmittel lassen sich besonders gut aus den Haaren ausspülen und führen zu besonders guten Haarpflegeergebnissen (insbesondere im Hinblick auf Kämmbarkeit und Haarfülle) ohne Auftreten einer Überkonditionierung (ölige, beschwerte Haare), wenn sie neben den zuvor genannten Wirkstoffen
- keine weiteren Fettphasenbestandteile - insbesondere keine Silikonöle und/oder Mineralöle und/oder von d)(i) verschiedene Fettsäureester - und
- keine nichtionischen, alkoxylierten Emulgatoren und/oder von b) verschiedenen polymeren Verdickungsmittel enthalten.

In einer weiteren bevorzugten Ausführungsform sind erfindungsgemäße Haarreinigungsmittel deshalb im Wesentlichen frei von Silikonen und/oder Mineralölen und/oder von d)(i) verschiedenen Fettsäureestern.

Erfindungsgemäß unter Wesentlichen frei" wird verstanden, dass die erfindungsgemäßen Haarreinigungsmittel weniger als 0,25 Gew.-%, bevorzugt weniger als 0,10 Gew.-% und insbesondere gar keine Silikone und/oder Mineralöle und/oder von d)(i) verschiedene Fettsäureester enthalten (bezogen auf das Gesamtgewicht der Haarreinigungsmittel).

Dabei gelten die zuvor genannten Mengenangaben sowohl für frei zugesetztes Silikon

und/oder Mineralöl und/oder von d)(i) verschiedenen Fettsäureester als auch für Silikone, und/oder Mineralöle und/oder von d)(i) verschiedene Fettsäureester, die gegebenenfalls als Nebenprodukt in Handelsprodukten enthalten sein können.

In einer weiteren bevorzugten Ausführungsform sind erfindungsgemäße Haarreinigungsmittel deshalb im Wesentlichen frei von nichtionischen, alkoxylierten Emulgatoren und/oder von b) verschiedenen polymeren Verdickungsmitteln.

Erfindungsgemäß unter "im Wesentlichen frei" wird verstanden, dass die erfindungsgemäßen Haarreinigungsmittel weniger als 0,10 Gew.-%, bevorzugt weniger als 0,05 Gew.-% und insbesondere gar keine nichtionischen, alkoxylierten Emulgatoren und/oder von b) verschiedenen polymeren Verdickungsmittel enthalten (bezogen auf das Gesamtgewicht der Haarreinigungsmittel).

Dabei gelten die zuvor genannten Mengenangaben sowohl für frei zugesetzte nichtionische, alkoxylierte Emulgatoren und/oder von b) verschiedene polymere Verdickungsmittel als auch für nichtionische, alkoxylierte Emulgatoren und/oder von b) verschiedene polymere Verdickungsmittel, die gegebenenfalls als Nebenprodukt in Handelsprodukten enthalten sein können.

Neben den zuvor genannten wesentlichen und fakultativen Bestandteilen können die erfindungsgemäßen Haarreinigungsmittel in einer weiteren bevorzugten Ausführungsform zur weiteren Steigerung der pflegenden Eigenschaften der Mittel mindestens einen weiteren haarkonditionierenden Wirkstoff enthalten, der ausgewählt sein kann aus der Gruppe der
- Proteinhydrolysate und/oder
- Vitamine.

Unter geeigneten Proteinhydrolysaten sind Produktgemische zu verstehen, die durch sauer, basisch oder enzymatisch katalysierten Abbau von Proteinen (Eiweißen) erhalten werden können. Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Der Gewichtsanteil des oder der Proteinhydrolysats(e) am Gesamtgewicht der Haarreinigungsmittel beträgt bevorzugt 0,01 bis 5 Gew.-%, mehr bevorzugt 0,025 bis 3 Gew.-% und insbesondere 0,05 bis 2 Gew.-%.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   > Vitamin B₁ (Thiamin)
   > Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere
      die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol. Der Gewichtsanteil des oder der Vitamins(e), Vitaminderivats(e), und/oder der Vitaminvorstufe(n) am Gesamtgewicht der Haarreinigungsmittel beträgt bevorzugt 0,001 bis 2 Gew.-%, besonders bevorzugt 0,005 bis 1 Gew.-% und insbesondere 0,01 bis 0,5 Gew.-%.

Es wurde festgestellt, dass die erfindungsgemäßen Haarreinigungsmittel auch für eine Anwendung als Antischuppenzubereitung geeignet sind.

Der Gewichtsanteil an Antischuppenmitteln am Gesamtgewicht der Haarreinigungsmittel kann bevorzugt 0,01 bis 10 Gew.-%, mehr bevorzugt 0,025 bis 7,5 Gew.-%, besonders bevorzugt 0,05 bis 5 Gew.-% und insbesondere 0,075 bis 3 Gew.-% betragen.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Bevorzugt sind Climbazol, Zink Pyrithion und Piroctone Olamine. Insbesondere bevorzugt ist Zink Pyrithion.

Weitere Wirk-, Hilfs- und Zusatzstoffe, die in den erfindungsgemäßen Haarreinigungsmitteln bevorzugt enthalten sein können, sind beispielsweise:
- Pflanzenextrakte,
- Feuchthaltemittel,
- Parfums,
- UV-Filter,
- Strukturanten wie Maleinsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Wirkstoffe wie Bisabolol und/oder Allantoin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure,
- Zusätzliche Viskositätsregler wie Salze (NaCl).

Durch die Kombination der Wirkstoffe a) bis c) in einem kosmetischen Träger - vorzugsweise in Wasser - können transparente Haarpflegeshampoos zur Verfügung gestellt werden, welche sehr gut ausspülbar sind und welche die Haare hervorragend reinigen, gut hautverträglich sind, sehr gut schäumen und den Haaren weiterhin verbesserte Pflegeigenschaften, insbesondere verbesserte Kämmbarkeit und mehr Haarfülle verleihen.

Durch die besonders gute Ausspülbarkeit der erfindungsgemäßen Haarreinigungsmittel konnte der für die Haarreinigung benötigte Wasserverbrauch im Durchschnitt bis zu 43% reduziert werden (gegenüber marktüblichen Shampoos, welche andere kationischen Polymere enthalten).

Erfindungsgemäß besonders bevorzugte Haarreinigungsmittel sind demnach transparent.

Unter "Transparenz" im Sinne der vorliegenden Erfindung werden erfindungsgemäße Haarreinigungsmittel verstanden, die einen NTU-Wert von 50 oder weniger, vorzugsweise von 30 oder weniger aufweisen, wobei die NTU-Wert-Bestimmung bei 20°C erfolgt.

In einer besonders bevorzugten Ausführungsform weisen die erfindungsgemäßen Haarreinigungsmittel nach der Lagerung für 12 Wochen bei 40°C noch immer einen NTU-Wert von 50 oder weniger, vorzugsweise von 30 oder weniger auf.

Ein zweiter Gegenstand der Erfindung ist kosmetische Verwendung des erfindungsgemäßen Haarreinigungsmittels zur Verbesserung der (insbesondere der zügigeren) Ausspülbarkeit sowie zur Verbesserung der Pflegeeigenschaften von Haaren wie (Nass)-Kämmbarkeit und Fülle. Bezüglich weiterer bevorzugter Ausführungsformen der erfindungsgemäßen Verwendung gilt mutatis mutandis das zu den erfindungsgemäßen Mitteln Gesagte.

Die folgenden Beispiele sollen den Gegenstand der vorliegenden Erfindung erläutern.

### Patentbeispiele:

a) Es wurden die folgenden erfindungsgemäßen Haarreinigungszusammensetzungen hergestellt, wobei die Zusammensetzungen 1-5, 16-20 und 31-35 Referenzbeispiele sind (die Mengenangaben beziehen sich auf Gew.-%):

| | **1** | **2** | **3** | **4** | **5** |
|---|---|---|---|---|---|
| Tensid* | 2,50 - 30,0 | 4,00 - 25,0 | 5,00 - 20,0 | 6,00 - 17,5 | 7,00 - 15,0 |
| kationisches Cassia-Polymer | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | | | | | |
|---|---|---|---|---|---|
| * anioniches, amphoteres, zwitterionisches und/oder nichtionisches | | | | | |

| | **6** | **7** | **8** | **9** | **10** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| kationisches Cassia-Polymer | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **11** | **12** | **13** | **14** | **15** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Kation. Cassia-Polymer | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **16** | **17** | **18** | **19** | **20** |
|---|---|---|---|---|---|
| Tensid* | 2,50 - 30,0 | 4,00 - 25,0 | 5,00 - 20,0 | 6,00 - 17,5 | 7,00 - 15,0 |
| Cassia Hydroxypropyltrimonium Chloride | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | | | | | |
|---|---|---|---|---|---|
| *anioniches, amphoteres, zwitterionisches und/oder nichtionisches | | | | | |

| | **21** | **22** | **23** | **24** | **25** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| Cassia Hydroxypropyltrimonium Chloride | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **26** | **27** | **28** | **29** | **30** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Amidoamin der Formel (I) | 0,01 - 2,00 | 0,025 - 1,75 | 0,05 - 1,50 | 0,10 - 1,25 | 1,00 |
| Wasser und ggfs. weitere Hilfs- und Zusatzstoffe | ad 100 | ad 100 | ad 100 | ad 100 | ad 100 |

| | **31** | **32** | **33** | **34** | **35** |
|---|---|---|---|---|---|
| Tensid* | 2,50 - 30,0 | 4,00 - 25,0 | 5,00 - 20,0 | 6,00 - 17,5 | 7,00 - 15,0 |
| Kation. Cassia-Polymer b) | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | | | | | |
|---|---|---|---|---|---|
| * anioniches, amphoteres, zwitterionisches und/oder nichtionisches | | | | | |

| | **36** | **37** | **38** | **39** | **40** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| Kation. Cassia-Polymer b) | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **41** | **42** | **43** | **44** | **45** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Kation. Cassia-Polymer b) | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **46** | **47** | **48** | **49** | **50** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Wasser und ggfs. weitere Hilfs- und Zusatzstoffe | ad 100 | ad 100 | ad 100 | ad 100 | ad 100 |

| | **51** | **52** | **53** | **54** | **55** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Ester d)(i) | 0,10 - 5,00 | 0,15 - 4,50 | 0,20 - 4,00 | 0,25 - 3,50 | 0,30 - 3,00 |
| pflanzliches Öl d)(ii) | 0,005 - 1,00 | 0,01 - 0,90 | 0,015 - 0,80 | 0,02 - 0,70 | 0,025 - 0,60 |
| Wasser und ggfs. weitere Hilfs- und Zusatzstoffe | ad 100 | ad 100 | ad 100 | ad 100 | ad 100 |

| | **56** | **57** | **58** | **59** | **60** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Glyceryl Oleate | 0,10 - 5,00 | 0,15 - 4,50 | 0,20 - 4,00 | 0,25 - 3,50 | 0,30 - 3,00 |
| pflanzliches Öl d)(ii) | 0,005 - 1,00 | 0,01 - 0,90 | 0,015 - 0,80 | 0,02 - 0,70 | 0,025 - 0,60 |
| Wasser und ggfs. weitere Hilfs- und Zusatzstoffe | ad 100 | ad 100 | ad 100 | ad 100 | ad 100 |

| | **61** | **62** | **63** | **64** | **65** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Glyceryl Oleate | 0,10 - 5,00 | 0,15 - 4,50 | 0,20 - 4,00 | 0,25 - 3,50 | 0,30 - 3,00 |
| Arganöl, Avocadoöl, Jojobaöl, Mandelöl, Rapsöl und/oder Sonnenblumenöl | 0,005 - 1,00 | 0,01 - 0,90 | 0,015 - 0,80 | 0,02 - 0,70 | 0,025 - 0,60 |
| Wasser und ggfs. weitere Hilfs- und Zusatzstoffe | ad 100 | ad 100 | ad 100 | ad 100 | ad 100 |

| | **66** | **67** | **68** | **69** | **70** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Ester d)(i) | 0,10 - 5,00 | 0,15 - 4,50 | 0,20 - 4,00 | 0,25 - 3,50 | 0,30 - 3,00 |
| pflanzliches Öl d)(ii) | 0,005 - 1,00 | 0,01 - 0,90 | 0,015 - 0,80 | 0,02 - 0,70 | 0,025 - 0,60 |
| Hydroxycarbonsäure | 0,10 - 5,00 | 0,20 - 4,00 | 0,30 - 3,00 | 0,40 - 2,00 | 0,50 - 1,00 |
| Wasser und ggfs. weitere Hilfs- und Zusatzstoffe | ad 100 | ad 100 | ad 100 | ad 100 | ad 100 |

| | **71** | **72** | **73** | **74** | **75** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Ester d)(i) | 0,10 - 5,00 | 0,15 - 4,50 | 0,20 - 4,00 | 0,25 - 3,50 | 0,30 - 3,00 |
| pflanzliches Öl d)(ii) | 0,005 - 1,00 | 0,01 - 0,90 | 0,015 - 0,80 | 0,02 - 0,70 | 0,025 - 0,60 |
| Milchsäure und/oder Zitronensäure | 0,10 - 5,00 | 0,20 - 4,00 | 0,30 - 3,00 | 0,40 - 2,00 | 0,50 - 1,00 |
| Wasser und ggfs. weitere Hilfs- und Zusatzstoffe | ad 100 | ad 100 | ad 100 | ad 100 | ad 100 |

| | **76** | **77** | **78** | **79** | **80** |
|---|---|---|---|---|---|
| Natriumlaurylethersulfat | 2,50 - 20,0 | 3,00 - 18,0 | 4,00 - 16,0 | 5,00 - 14,0 | 6,00 - 12,5 |
| Cocamidopropylbetain und/oder Disodium Cocoamphodiacetate | 1,00 - 10,00 | 1,20 - 9,00 | 1,40 - 8,00 | 1,60 - 7,00 | 1,80 - 6,00 |
| Cassia Hydroxypropyltrimonium Chloride | 0,01 - 1,00 | 0,02 - 0,90 | 0,03 - 0,80 | 0,04 - 0,70 | 0,05 - 0,60 |
| Behenamidopropyl Dimethylamine | 0,01 - 2,00 | 0,025 - 1,75 | 0,05 - 1,50 | 0,10 - 1,25 | 0,15 - 1,00 |
| Glyceryl Oleate | 0,10 - 5,00 | 0,15 - 4,50 | 0,20 - 4,00 | 0,25 - 3,50 | 0,30 - 3,00 |
| Arganöl | 0,005 - 1,00 | 0,01 - 0,90 | 0,015 - 0,80 | 0,02 - 0,70 | 0,025 - 0,60 |
| Milchsäure und/oder Zitronensäure | 0,10 - 5,00 | 0,20 - 4,00 | 0,30 - 3,00 | 0,40 - 2,00 | 0,50 - 1,00 |
| Wasser und ggfs. weitere Hilfs- und Zusatzstoffe | ad 100 | ad 100 | ad 100 | ad 100 | ad 100 |

b) Beurteilung der Auswaschbarkeit erfindungsgemäßer (I) und nicht erfindungsgemäßer Haarreinigungsmittel (II, III)

| | **I** | **II** | **III** |
|---|---|---|---|
| Natriumlaurylethersulfat | 10,00 | 10,00 | 9,00 |
| Disodium Cocoamphodiacetate | 0,60 | 0,60 | 0,60 |
| Cocamidopropylbetaine | 1,80 | 1,80 | 2,00 |
| Cassia Hydroxypropyltrimonium Chloride | 0,10 | | |
| Hydroxypropyl Guar Hydroxypropyltrimonium Chloride | | 0,10 | |
| Polyquaternium-10 | | | 0,10 |
| Behenamidopropyl Dimethylamine | 0,40 | 0,40 | |
| Arganöl | 0,10 | 0,10 | 0,10 |
| Glyceryl Oleate | 0,60 | 0,60 | |
| Coco Glucoside | 0,60 | 0,60 | |
| PEG-7 Glyceryl Cocoate | | | 0,50 |
| Parfum | 0,30 | 0,30 | 0,30 |
| pH-Stellmittel (Milchsäure und/oder Zitronensäure), Konservierungsmittel, NaCl | q.s. | q.s. | q.s. |
| Wasser | ad 100 | ad 100 | ad 100 |
| pH | 4,0 - 5,0 | 4,0 - 5,0 | 4,0 - 5,0 |

### Verwendete Materialien:

Verwendeter Mannequin-Kopf: Emma, medium Haar; Verwendeter Duschkopf: GROHE Rainshower Solo 100; Verwendeter Durchflusskonstanthalter (DFK): 5,7h I/min (entsprechend der Restriktionen des maximalen Wasserdurchflusses in den USA), der Wasserhahn wird stets vollständig geöffnet, um konstanten Durchfluss zu gewährleisten; Wassertemperatur: 38°C;
Abstand Kopf-Brause: 20 cm (markierte Stelle an der Duschhalterung);
Hintergrund: gemessen wird die Trübung des Waschwassers (Tenside, Trübungsmittel etc. bilden im Wasser Koazervate, die das Waschwasser trüben)

Durchführung der Messungen:
1. Vorbehandlung: Mannequin-Kopf kämmen und ggf. nass machen
2. Standardisiertes Auftragen des Shampoos: Anwendungsmenge: 10 g des jeweiligen Shampoos einwiegen, auf den Mannequin-Kopf auftragen und für 60 sec einmassieren
3. Start der Trübungsaufnahme im Prüfstand Brausekopf mit "Start Messung"
4. Öffnen des Wasserhahns bis zum Anschlag
5. Aufnahme der Messdaten bis zu einer Trübung von unter 50 NTU bzw. für etwa 360 sec bis kein Schaum mehr auf Mannequin und in der Duschkabine zu erkennen ist, mögliche Schaumreste im "Abflusslabyrinth", die zu einer Resttrübung führen können
6. Beenden der Trübungsaufnahme mit "Stopp Messung"
7. Exportieren der Daten mit "Export" als .csv Datei, die später als Excel-Datei gespeichert werden kann
8. Nach Punkt 6. Das Wasser noch ein wenig laufen lassen und den Schaum im "Abflusslabyrinth" mit Gummiwischer abschöpfen, damit für eine weitere Messung keine Tensidreste im "Abflusslabyrinth" verbleiben

Reproduzierbarkeit: Zu Zwecken der Reproduzierbarkeit werden Messungen mit einem Shampoo in Fünffachbestimmung durchgeführt, um die Standardabweichung und die durchschnittliche Dauer des Auswaschvorgangs bestimmen zu können.

Definition "Ausgewaschen": Als ausgewaschen gilt ein Shampoo, sobald die Trübung konstant unter 50 NTU bleibt. Bei dieser verbleibenden Resttrübung verbleibt kein Shampoo in den Haaren und der Duschkabine.

Mechanik: kein Einwirken von Mechanik auf den Kopf, ausschließlich Druck durch den Wasserstrahl, daher ist die Auswaschzeit der Shampoos in der Duschkabine nicht direkt auf die im Haarstudio bzw. in der Realität übertragbar. Vergleiche zwischen Shampoos können im Vergleich allerdings gemacht werden

Vorgehensweise Datenaufbereitung:
1.csv-Datei in .xlsx-Datei umwandeln
2. Wiederholungsmessungen in Excel-Datei speichern
3. Startpunkt des Auswaschvorgangs in der Datenreihe markieren (aufgrund von fehlendem Durchfluss ist die gemessene Trübung zum Zeitpunkt des Messstarts ohne Wasserdurchfluss eher hoch, fällt bei der ersten Bewegung des Wassers im "Auswaschlabyrinth" wieder und der Zeitpunkt, an dem die Trübung wieder zunimmt, wird als Startwert der Messung (t=0) definiert
4. Durchschnittswerte für die Datenreihen ab t=0 bestimmen (bei Fünffachbestimmung Mittelwert aus fünf Messwiederholungen)
5. Standardabweichung für die Datenreihen ab t=0 bestimmen
6. Graph aus den Einzelmessungen und den Durchschnittswerten erstellen, um Abweichungen und den Verlauf des Auswaschens darzustellen
7. Definierten Endpunkt in der Datenreihe markieren (Endpunkt siehe oben, wenn die Trübung konstant <50 NTU),
8. Zeitpunkt t (<50 NTU) in sec. für die jeweilige Datenreihe bestimmen
Vorgehensweise Ergebnisauflistung:
1. Angabe von Mannequin; Anwendungsmenge; Duschkopf; Abstand Kopf-Brause; Haartyp; Shampoo; Kürzel; Optiva-Nr.; t(<50 NTU)in sec. [hier wird jeweils der Zeitpunkt gewählt, bei dem die durchschnittliche Trübung aus der Mehrfachbestimmung <50 NTU ist]; Standard-abweichung s; Verwendetes Wasser in [I] sowie Energieverbrauch Erhitzen Wasser in Wh
2. Angabe der Einzelwerte der Mehrfachbestimmung bei t(<50 NTU) der durchschnittlichen Trübung [bei Fünffachbestimmung fünf Trübungswerte zu demselben Zeitpunkt t]
3. Errechnung des Durchschnitts und der Standardabweichung
4. Angabe der Zeit in [sec], bei der die Trübung der Einzelmessungen konstant bei <50 NTU
5. Errechnung des Durchschnitts und der Standardabweichung der durchschnittlich verstrichenen Zeit bis zu einer Trübung <50 NTU

Vorgehensweise Datenvergleich:
Vergleich der Zeit t bei T > 50 NTU (im Durchschnitt) inklusive der Standardabweichung t-Test: Vergleich der Zeit t in [sec], bei der die Trübung <50 NTU beträgt (aus den Einzelmessungen, bei Fünffachbestimmung fünf Einzelseiten)

### Ergebnis Auswaschbarkeit:

| | **I** | **II** | **III** |
|---|---|---|---|
| **% Unterschied der Zeit t bei T <50FNU zum Standard (III)** | -46 | -34 | 0 |

c) Beurteilung der Haarvolumens von Haaren, die mit einem erfindungsgemäßen (I) und einem nicht erfindungsgemäßen Haarreinigungsmittel (II) gewaschen wurden

### Probandentext:

Frage: wie zufrieden sind Sie mit der Fülle Ihrer Haare nach Anwendung des Shampoos?

| **Shampoo** | **Bewertung** | | | | | | | **Zahl Probanden** |
|---|---|---|---|---|---|---|---|---|
| | 1 | 2 | 3 | 4 | 5 | 6 | Durchschnitt | |
| I | 0 | 1 | 1 | 3 | 4 | 1 | 4,3 | 10 |
| **II** | 0 | 1 | 4 | 3 | 2 | 0 | 3,6 | 10 |

| | | | | | | | | |
|---|---|---|---|---|---|---|---|---|
| 1: überhaupt nicht zufrieden 6: vollkommen zufrieden Befragung je Variante von 10 Verwendern mit normalem Haar* nach zwei Wochen Verwendung des jeweiligen Produktes. Die Produkte wurden neutral verpackt und entsprechend kodiert. *Selbsteinschätzung der Probanden | | | | | | | | |

d) Beurteilung der Nasskämmbarkeit von Haaren, die mit einem erfindungsgemäßen (I) und einem nicht erfindungsgemäßen Haarreinigungsmittel (II) gewaschen wurden:
1. Vorbehandlung von Haarsträhnen
Für beide Zusammensetzungen I und II wurden jeweils 10 Haarsträhnen verwendet.
Diese wurden auf dieselbe Art und Weise gebleicht und anschließend bei 25°C und 25% relativer Luftfeuchtigkeit für 48 Stunden gelagert.
Als Standard wurden gebleichte Haarsträhnen verwendet, die 5 Minuten vor den
Kämmbarkeitsmessungen mit Wasser durchtränkt wurden.

2. Auftragen der Zusammensetzungen I und II
0,25 g (pro g Haar) des jeweiligen Shampoos I und II wurden auf die nassen Haarsträhnen aufgetragen. Nach 2 Minuten wurden die Strähnen unter Standardbedingungen (mit Leitungswasser bei 32°C; 0,5 I pro Minute) für 18 Sekunden gespült und währenddessen automatisch gekämmt.
Dieser Vorgang wurde je Haarsträhne und aufgetragener Zusammensetzung jeweils einmal wiederholt.

3. Messung der Nasskämmbarkeit
Jede Haarsträhne wurde vor der Messung nochmal für 2 Sekunden gekämmt und befeuchtet.
Nach dreimaligem Kämmvorgängen (pre-combing strokes) wurden 10 Kämmdurchgänge durchgeführt, während denen die jeweiligen Haarsträhnen langsam rotiert wurden. Dabei wurde die durchschnittliche Arbeit während des Kämmens bestimmt. Die folgenden Ergebnisse wurden erzielt:

| | **I** | **II** |
|---|---|---|
| **Änderung der Nasskämmbarkeit gegenüber gebleichten Haarsträhnen (Standard)** | 53 | 26 |

## Patentansprüche

1. Haarreinigungsmittel, enthaltend (bezogen auf das Gewicht des gesamten Mittels)
a) mindestens ein Tensid, ausgewählt aus
ai) 2,5 bis 20 Gew.-% mindestens eines anionischen Tensids und
aii) 1,0 bis 10 Gew.-% mindestens eines amphoteren und/oder zwitterionischen Tensids,
b) 0,01 bis 1,00 Gew.-% mindestens eines kationischesn Cassia-Polymers und
c) 0,01 bis 2,00 Gew.-% mindestens eines Amidoamins der Formel (I), 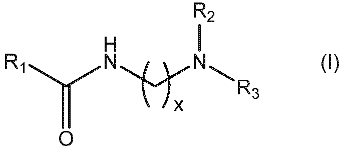 worin
R₁ für einen gesättigten oder ungesättigten, verzweigten oder unverzweigten Alkylrest mit 19 bis 30 Kohlenstoffatomen steht,
R₂ und R₃ unabhängig voneinander für Wasserstoff oder für eine C₁-C₄-Alkylgruppe stehen, und
x für eine ganze Zahl von 2 bis 6 steht.

2. Haarreinigungsmittel nach Anspruch 1, wobei das kationische Cassia-Polymer b) mit Hydroxy-C₁-C₃-alkyl-trialkylammonium-Gruppen kationisch modifiziert ist, vorzugsweise wobei das kationische Cassia-Polymer ausgewählt ist aus unter der INCI-Bezeichnung Cassia Hydroxypropyltrimonium Chloride bekannten Verbindungen.

3. Haarreinigungsmittel nach einem der vorhergehenden Ansprüche wobei das mindestens eine Amidoamin c) ausgewählt ist aus Verbindungen der Formel (I), worin
- R₁ für einen Alkenylrest mit 20 bis 24 Kohlenstoffatomen steht,
- R₂ und R₃ für Methylgruppen stehen, und
- x für die Zahlen 2, 3 oder 4 steht.

4. Haarreinigungsmittel nach Anspruch 3, wobei das mindestens eine Amidoamin c) ausgewählt ist aus unter der INCI-Bezeichnung Behenamidopropyl Dimethylamine bekannten Amidoaminen.

5. Haarreinigungsmittel nach einem der vorhergehenden Ansprüche, enthaltend mindestens ein natürliches, mineralisches und/oder synthetisches Öl in einem Gewichtsanteil von 0,1 bis 10 Gew.-% am Gesamtgewicht des Haarreinigungsmittels.

6. Haarreinigungsmittel nach Anspruch 5, enthaltend
(i) mindestens einen Mono- und/oder Diester von Glycerin und einer gesättigten oder ungesättigten, verzweigten oder unverzweigten Carbonsäure mit 18 bis 30 Kohlenstoffatomen, und
(ii) mindestens ein pflanzliches Öl.

7. Haarreinigungsmittel nach Anspruch 6, wobei
- der mindestens eine Mono- und/oder Diester (i) ausgewählt ist aus Mono- und/oder Diestern von Glycerin und ungesättigten Carbonsäuren mit 18 bis 24 Kohlenstoffatomen, vorzugsweise aus unter der INCI-Bezeichnung Glyceryl Oleate bekannten Glycerinestern,
- das mindestens eine pflanzliche Öl (ii) ausgewählt ist aus Arganöl, Avocadoöl, Sonnenblumenöl, Jojobaöl, Rapsöl und/oder Mandelöl, vorzugsweise aus Arganöl.

8. Haarreinigungsmittel nach einem der vorhergehenden Ansprüche, enthaltend (bezogen auf das Gewicht des gesamten Mittels) 0,01 bis 5,00 Gew.-% mindestens einer Hydroxycarbonsäure, vorzugsweise Milchsäure und/oder Zitronensäure und/oder Glutaminsäure und/oder Bernsteinsäure.

9. Haarreinigungsmittel nach einem der vorhergehenden Ansprüche, welches weniger als 0,10 Gew.-%, mehr bevorzugt weniger als 0,05 Gew.-% und insbesondere gar keine nichtionischen, alkoxylierten Emulgatoren und/oder von b) verschiedenen polymeren Verdickungsmittel enthält (bezogen auf das Gesamtgewicht des Haarreinigungsmittels).

10. Haarreinigungsmittel nach einem der vorhergehenden Ansprüche, welches weniger als 0,25 Gew.-%, mehr bevorzugt weniger als 0,10 Gew.-% und insbesondere gar keine Silikone und/oder Mineralöle und/oder von d)(i) verschiedene Fettsäureester enthält (bezogen auf das Gesamtgewicht des Haarreinigungsmittels).

11. Haarreinigungsmittel nach einem der vorhergehenden Ansprüche, welches transparent ist.

12. Haarreinigungsmittel nach einem der vorhergehenden Ansprüche, welches (bezogen auf das Gewicht des gesamten Mittels) mindestens 75 Gew.-% Wasser, vorzugsweise mindestens 80 Gew.-% Wasser enthält.

13. Kosmetische Verwendung eines Haarreinigungsmittels nach einem Ansprüche 1 bis 12 zur Verbesserung der (insbesondere zügigeren) Ausspülbarkeit des Haarreinigungsmittels sowie zur Verbesserung der (Nass)-Kämmbarkeit und der Fülle der behandelten Haare.

## Claims

1. Hair cleansing agent containing (based on the weight of the entire agent)
a) at least one surfactant selected from
ai) 2.5 to 20% by weight of at least one anionic surfactant and
aii) 1.0 to 10% by weight of at least one amphoteric and/or zwitterionic surfactant,
b) 0.01 to 1.00% by weight of at least one cationic cassia polymer and
c) 0.01 to 2.00% by weight of at least one amidoamine of formula (I), 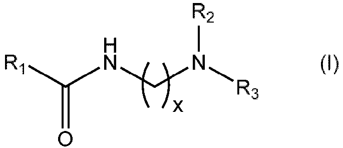 where
_{R1} is a saturated or unsaturated, branched or unbranched alkyl radical with 19 to 30 carbon atoms,
_{R2} and _{R3} independently represent hydrogen or a _{C1-C4} alkyl group, and
x is an integer from 2 to 6.

2. Hair cleansing agent according to claim 1, wherein the cationic cassia polymer b) is cationically modified with hydroxy-C1-C3-alkyl-trialkylammonium groups, preferably wherein the cationic cassia polymer is selected from compounds known under the INCI designation Cassia Hydroxypropyltrimonium Chloride.

3. Hair cleansing agent according to one of the preceding claims, wherein the at least one amidoamine c) is selected from compounds of formula (I), wherein
- _{R1} represents an alkenyl radical with 20 to 24 carbon atoms,
- _{R2} and _{R3} represent methyl groups, and
- x stands for the numbers 2, 3 or 4.

4. Hair cleansing agent according to claim 3, wherein the at least one amidoamine is selected from amidoamines known under the INCI designation Behenamidopropyl Dimethylamine.

5. Hair cleansing agent according to one of the preceding claims, containing at least one natural, mineral and/or synthetic oil in a proportion of 0.1 to 10% by weight of the total weight of the hair cleansing agent.

6. Hair cleansing agent according to claim 5, containing
(i) at least one mono- and/or diester of glycerin and a saturated or unsaturated, branched or unbranched carboxylic acid with 18 to 30 carbon atoms, and
(ii) at least one vegetable oil.

7. Hair cleansing agent according to claim 6, wherein
- the at least one mono- and/or diester (i) is selected from mono- and/or diesters of glycerin and unsaturated carboxylic acids with 18 to 24 carbon atoms, preferably from glycerin esters known under the INCI designation glyceryl oleate,
- the at least one vegetable oil (ii) is selected from argan oil, avocado oil, sunflower oil, jojoba oil, rapeseed oil and/or almond oil, preferably from argan oil.

8. Hair cleansing agent according to one of the preceding claims, containing (based on the weight of the entire agent) 0.01 to 5.00% by weight of at least one hydroxycarboxylic acid, preferably lactic acid and/or citric acid and/or glutamic acid and/or succinic acid.

9. Hair cleansing agent according to one of the preceding claims, which contains less than 0.10% by weight, more preferably less than 0.05% by weight and, in particular, no nonionic, alkoxylated emulsifiers and/or of b) various polymeric thickening agents (based on the total weight of the hair cleansing agent).

10. Hair cleansing agent according to one of the preceding claims, which contains less than 0.25% by weight, more preferably less than 0.10% by weight and, in particular, no silicones and/or mineral oils and/or d)(i) various fatty acid esters (based on the total weight of the hair cleansing agent).

11. Hair cleansing agent according to one of the preceding claims, which is transparent.

12. Hair cleansing agent according to one of the preceding claims, which contains at least 75% by weight of water, preferably at least 80% by weight of water (based on the weight of the entire agent).

13. Cosmetic use of a hair cleansing agent according to claims 1 to 12 for improving the (in particular faster) rinsability of the hair cleansing agent and for improving the (wet) combability and fullness of the treated hair.

## Revendications

1. Produit nettoyant pour cheveux contenant (par rapport au poids total du produit)
a) au moins un tensioactif choisi parmi
ai) 2,5 à 20 % en poids d'au moins un tensioactif anionique et
aii) 1,0 à 10 % en poids d'au moins un tensioactif amphotère et/ou zwitterionique,
b) 0,01 à 1,00 % en poids d'au moins un polymère cationique de type cassia et
c) 0,01 à 2,00 % en poids d'au moins une amidoamine de formule (I), 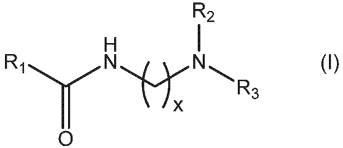 dans laquelle
R₁ représente un radical alkyle saturé ou insaturé, ramifié ou non ramifié, comportant 19 à 30 atomes de carbone,
R₂ et R₃ représentent indépendamment l'un de l'autre un atome d'hydrogène ou un groupe alkyle en C₁ -C₄, et
x représente un nombre entier compris entre 2 et 6.

2. Agent nettoyant pour cheveux selon la revendication 1, dans lequel le polymère cationique de cassia b) est modifié cationiquement par des groupes hydroxy-C₁ -C₃ -alkyl-trialkylammonium, de préférence le polymère cationique de cassia étant choisi parmi les composés connus sous la désignation INCI Cassia Hydroxypropyltrimonium Chloride.

3. Produit nettoyant pour cheveux selon l'une des revendications précédentes, dans lequel la au moins une amidoamine c) est choisie parmi les composés de formule (I) dans laquelle
- R₁ représente un radical alcényle ayant de 20 à 24 atomes de carbone,
- R₂ et R₃ représentent des groupes méthyle, et
- x représente les chiffres 2, 3 ou 4.

4. Produit nettoyant pour cheveux selon la revendication 3, dans lequel la au moins une amidoamine c) est choisie parmi les amidoamines connues sous la désignation INCI Behenamidopropyl Dimethylamine.

5. Produit nettoyant pour cheveux selon l'une des revendications précédentes, contenant au moins une huile naturelle, minérale et/ou synthétique dans une proportion en poids de 0,1 à 10 % du poids total du produit nettoyant pour cheveux.

6. Produit nettoyant pour cheveux selon la revendication 5, contenant
(i) au moins un mono- et/ou diester de glycérine et d'un acide carboxylique saturé ou insaturé, ramifié ou non ramifié, comportant 18 à 30 atomes de carbone, et
(ii) au moins une huile végétale.

7. Agent nettoyant pour cheveux selon la revendication 6, dans lequel
- le ou les mono- et/ou diesters (i) sont choisis parmi les mono- et/ou diesters de glycérine et d'acides carboxyliques insaturés comportant 18 à 24 atomes de carbone, de préférence parmi les esters de glycérine connus sous la désignation INCI « Glyceryl Oleate »,
- la au moins une huile végétale (ii) étant choisie parmi l'huile d'argan, l'huile d'avocat, l'huile de tournesol, l'huile de jojoba, l'huile de colza et/ou l'huile d'amande, de préférence parmi l'huile d'argan.

8. Produit nettoyant pour cheveux selon l'une des revendications précédentes, contenant (par rapport au poids total du produit) 0,01 à 5,00 % en poids d'au moins un acide hydroxycarboxylique, de préférence l'acide lactique et/ou l'acide citrique et/ou l'acide glutamique et/ou l'acide succinique.

9. Produit nettoyant pour cheveux selon l'une des revendications précédentes, qui contient moins de 0,10 % en poids, de préférence moins de 0,05 % en poids et en particulier pas du tout d'émulsifiants non ioniques alcoxylés et/ou de b) différents épaississants polymères (par rapport au poids total du produit nettoyant pour cheveux).

10. Produit nettoyant pour cheveux selon l'une des revendications précédentes, qui contient moins de 0,25 % en poids, de préférence moins de 0,10 % en poids et en particulier pas du tout de silicones et/ou d'huiles minérales et/ou de d)(i) différents esters d'acides gras (par rapport au poids total du produit nettoyant pour cheveux).

11. Produit nettoyant pour cheveux selon l'une des revendications précédentes, qui est transparent.

12. Produit nettoyant pour cheveux selon l'une des revendications précédentes, qui contient (par rapport au poids total du produit) au moins 75 % en poids d'eau, de préférence au moins 80 % en poids d'eau.

13. Utilisation cosmétique d'un produit nettoyant pour cheveux selon l'une des revendications 1 à 12 pour améliorer la rinçabilité (en particulier plus rapide) du produit nettoyant pour cheveux ainsi que la facilité de coiffage (sur cheveux mouillés) et la densité des cheveux traités.
